# EUROPEAN PATENT APPLICATION

(11) **EP 4 706 655 A1**
(43) Date of publication of application: **11.03.2026**
(21) Application number: 24800123.2
(22) Date of filing: 01.05.2024
(51) Int. Cl.: A61K 31/496, A61K 31/05, A61P 21/00, A61P 25/00, A61P 25/28, A61P 43/00

(54) **PREVENTIVE OR THERAPEUTIC AGENT FOR NEURODEGENERATIVE DISEASE**

(30) Priority: 02.05.2023 WO PCT/JP2023/017175
(71) Applicant: Medilabo RFP, Inc., Kyoto-shi, Kyoto 604-8136 (JP); University Public Corporation Osaka, Osaka-shi, Osaka 5360025 (JP)
(72) Inventor: KUMAGAI, Toru, Kyoto-shi, Kyoto 604-8136 (JP); TOMIYAMA, Takami, Sakai-shi, Osaka 599-8531 (JP); UMEDA, Tomohiro, Sakai-shi, Osaka 599-8531 (JP)
(74) Representative: Müller-Boré & Partner Patentanwälte PartG mbB
(86) International application number: PCT/JP2024/016800
(87) International publication number: WO 2024/228392

(57) **Abstract**

The purpose of the present invention is to provide a medicinal agent that exhibits the effect of inhibiting aggregation of a causative protein of an HRE-related neurodegenerative disease such as ALS. According to the present invention, rifampicin or a rifampicin compound selected from the group consisting of rifampicin, a derivative thereof, and a salt of rifampicin or the derivative and/or resveratrol or a resveratrol compound selected from the group consisting of resveratrol and a derivative thereof is an active ingredient of a preventive or therapeutic agent for a neurodegenerative disease caused by TDP-43 accumulation, or an active ingredient of a preventive or therapeutic agent for ALS.

## Description

### Technical Field

The present invention relates to a pharmaceutical product useful for preventing or treating a neurodegenerative disease.

### Background Art

Frontotemporal dementia (FTD) is a neurodegenerative disease that causes impairment mainly of cognitive function, and amyotrophic lateral sclerosis (ALS) is a neurodegenerative disease that causes impairment mainly of motor function. The number of patients with FTD (prevalence) is estimated to be 15 to 22 in 100,000, meanwhile the number of patients with ALS is estimated to be 5 to 6 in 100,000. About 30% of FTD and 5 to 10% of ALS are familial, i.e. hereditary, and both the diseases share some of the same causative genes. The most frequent genetic cause is a mutation in which a hexanucleotide (GGGGCC) repeat present in an untranslated region of the C9orf72 gene is abnormally extended (hexanucleotide repeat expansion, HRE) (NPL 1). The GGGGCC sequence in healthy individuals is repeated 30 times or less, whereas the sequence in patients is repeated 100 to 5000 times (NPL 2).

As a mechanism by which HRE causes disease, loss-of-function of C9orf72 protein and acquisition of toxicity of HRE-derived RNA or protein have been proposed (NPLs 3 and 4). In a first mechanism, HRE probably forms a G-quadruplex structure in the promoter region and functions as a steric block to the transcription mechanism, thereby inhibiting transcription of the C9orf72 gene (NPL 5). The C9orf72 protein is expressed in the brain, spinal cord, and immune cells, and has been reported to regulate autophagy and vesicle transport (NPL 4). A decreased expression of C9orf72 results in haploinsufficiency, which leads to autophagy dysfunction (NPL 6) and neurodegeneration (NPL 7). In a second mechanism, HRE-derived sense and antisense RNAs form a hairpin structure with the G-quadruplex (NPLs 8 and 9). These structures sequester RNA-binding proteins such as heteronuclear ribonucleoprotein (hnRNP) H in inclusions, i.e. RNA foci, in the nucleus and sometimes in the cytoplasm (NPLs 9 to 11). It is considered that the sequestering inhibits RNA processing, leading to RNA-mediated cytotoxicity (NPL 12). In a third mechanism, dipeptide repeat proteins (DPRs) such as poly-GA, poly-GP, poly-GR, poly-AP, and poly-PR are synthesized from HRE-derived sense and antisense RNAs by repeat-associated non-ATG (RAN) translation (NPLs 13 to 17).

These DPRs self-aggregate to form inclusions in the cytoplasm and sometimes in the nucleus, and are associated with p62: autophagy- or ubiquitin-proteasome-related protein (NPLs 13,14,16,18, and 19). Furthermore, HRE-derived RNAs and DPRs promote aberrant cytoplasmic liquid-liquid phase separation (LLPS) to form membraneless organelles called stress granules, in which RNA, RNA-binding proteins such as TDP-43, and the translation machinery are condensed (NPLs 20 to 22). Disease-related LLPS has been first suggested for FUS proteins (NPLs 23 and 24), but now many proteins with low complexity domains (LCD) including TDP-43 and other RNA-binding proteins have been involved in this phenomenon (NPL 25). Furthermore, HRE-derived RNAs and DPRs inhibit the nuclear-cytoplasm transport by forming nuclear pore complexes (NPLs 22, 26, and 27). These alterations accelerate the cytoplasmic accumulation of TDP-43 and its self-aggregation in stress granules, leading to the formation of cytoplasmic TDP-43 inclusions and the depletion of nuclear TDP-43, which causes loss-of-function of the protein (NPLs 22 and 28). The presence of cytoplasmic inclusions of phosphorylated TDP-43 (pTDP-43) and loss of the nuclear localization of TDP-43 are characteristics of C9orf72-linked FTD/ALS (NPL 28). Furthermore, TDP-43 forms toxic oligomers within cells in the brains of FTD and ALS patients (NPLs 28 to 30). TDP-43 inclusions, but not RNA foci or DPR inclusions (NPLs 2 and 19), appear to be associated with neurodegeneration, suggesting that TDP-43 is an eventual effector of C9orf72 HRE mutations.

These studies suggest that inhibition of G-quadruplex formation and RAN translation enables C9orf72 related diseases to be prevented. RAN translation has been shown to be highly regulated by double-stranded RNA-dependent protein kinase (PKR), and the PKR is activated by disease relevant repeat expansion RNAs via phosphorylation of Thr446 and Thr451 residues (NPL 31). Furthermore, a therapeutic agent for type 2 diabetes: metformin has been reported to suppress RAN translation by suppressing PKR phosphorylation, thereby improving the phenotype of C9-500 mice (NPL 31). Consequently, it is considered that inhibition of PKR phosphorylation leads to inhibition of RAN translation.

### Citation List

### Non Patent Literature

- NPL 1:: Neuron 2011, 72, 245-256.
- NPL 2:: Curr. Opin. Neurobiol. 2016, 36, 99-106.
- NPL 3:: Nat.Rev. Neurol. 2018, 14, 544-558.
- NPL 4:: Front. Cell. Neurosci. 2021, 15, 661447.
- NPL 5:: Chem. Soc. Rev. 2008, 37, 1375-1384.
- NPL 6:: Autophagy 2021, 17, 3306-3322.
- NPL 7:: Nat. Med. 2018, 24, 313-325.
- NPL 8:: Nature 2014, 507, 195-200.
- NPL 9:: Elife 2016, 5, e17820.
- NPL 10:: Cell Rep. 2013, 5, 1178-1186.
- NPL 11:: Brain 2014, 137, 2040-2051.
- NPL 12:: Neurobiol. Dis. 2020, 145, 105055.
- NPL 13:: Neuron 2013, 77, 639-646.
- NPL 14:: Science 2013, 339, 1335-1338.
- NPL 15:: Acta Neuropathol. 2013, 126, 829-844.
- NPL 16:: Acta Neuropathol. 2013, 126, 881-893.
- NPL 17:: Proc. Natl. Acad. Sci. USA 2013, 110, E4968-E4977.
- NPL 18:: Acta Neuropathol. Commun. 2013, 1, 68.
- NPL 19:: Acta Neuropathol. 2014, 127, 347-357.
- NPL 20:: Cell Rep. 2017, 21, 3573-3584.
- NPL 21:: Biophys. J. 2020, 119, 843-851.
- NPL 22:: Front. Cell. Neurosci. 2021, 15, 664151.
- NPL 23:: Cell 2015, 162, 1066-1077.
- NPL 24:: Neuron 2015, 88, 678-690.
- NPL 25:: EMBO J. 2016, 35, 1603-1612.
- NPL 26:: Nature 2015, 525, 56-61.
- NPL 27:: Hum. Mol. Genet. 2017, 26, 790-800.
- NPL 28:: Front. Mol. Neurosci. 2019, 12, 25.
- NPL 29:: Nat. Commun. 2014, 5, 4824.
- NPL 30:: Neurobiol. Dis. 2020, 146, 105130.
- NPL 31:: Proc. Natl. Acad. Sci. USA 2020, 117, 18591-18599.

### Summary of Invention

### Technical Problem

An HRE-related neurodegenerative disease such as ALS still lacks effective therapies. When a medicinal agent that exhibits the effect of inhibiting aggregation of a causative protein (such as DPR or TDP-43) of the neurodegenerative disease is found, it can be a potential candidate for a preventive agent for HRE-related neurodegenerative disease.

So, the purpose of the present invention is to provide a medicinal agent that exhibits the effect of inhibiting aggregation of a causative protein of an HRE-related neurodegenerative disease such as ALS.

### Solution to Problem

The present inventors have administered rifampicin to FTD/ALS model mice produced by introducing a human full length C9orf72 gene that repeats a GGGGCC sequence about 500 times, and examined the influence of rifampicin on the formation of RNA foci and cytoplasmic inclusions composed of DPR or TDP-43 in the brain. They have found that nasal administration of rifampicin for 1 month results in prevention of HRE-related neuropathology (aggregation of DPR, TDP-43, etc.) and improvement of memory performance of FTD/ALS model mice at the age of 5.5 to 6 months. In addition, they have found that all of the single administration of rifampicin, combined administration of rifampicin and resveratrol, and single administration of resveratrol to the FTD/ALS model mice result in the effect of improving the cognitive function, and resveratrol has a remarkable effect of improving the cognitive function of the FTD/ALS model mice. The present invention has been completed by further conducting studies based on these findings.

That is, the present invention provides inventions of the following aspects.

Item 1. A preventive or therapeutic agent for a neurodegenerative disease caused by accumulation of TDP-43, including, as an active ingredient, a resveratrol compound selected from the group consisting of resveratrol and a derivative of resveratrol.

Item 2. A preventive or therapeutic agent for amyotrophic lateral sclerosis (ALS), including, as an active ingredient, a resveratrol compound selected from the group consisting of resveratrol and a derivative of resveratrol.

Item 3. A preventive or therapeutic agent for a neurodegenerative disease caused by C9orf72 gene abnormality and/or TDP-43 gene abnormality, including, as an active ingredient, a resveratrol compound selected from the group consisting of resveratrol and a derivative of resveratrol.

Item 4. The preventive or therapeutic agent according to any one of items 1 to 3, wherein the preventive or therapeutic agent is used for nasal administration.

Item 5. The preventive or therapeutic agent according to any one of items 1 to 4, wherein a dose of the resveratrol compound is 3.75 mg/kg·day or less.

Item 6. A preventive or therapeutic agent for a neurodegenerative disease caused by accumulation of TDP-43, including a combination of a rifampicin compound selected from the group consisting of rifampicin, a derivative of rifampicin, and a salt of rifampicin or the derivative, and a resveratrol compound selected from the group consisting of resveratrol and a derivative of resveratrol.

Item 7. A preventive or therapeutic agent for a neurodegenerative disease caused by accumulation of TDP-43, including, as an active ingredient, a rifampicin compound selected from the group consisting of rifampicin, a derivative of rifampicin, and a salt of rifampicin or the derivative.

Item 8. A preventive or therapeutic agent for amyotrophic lateral sclerosis (ALS), including, as an active ingredient, a rifampicin compound selected from the group consisting of rifampicin, a derivative of rifampicin, and a salt of rifampicin or the derivative.

Item 9. A preventive or therapeutic agent for a neurodegenerative disease caused by C9orf72 gene abnormality and/or TDP-43 gene abnormality, including, as an active ingredient, a rifampicin compound selected from the group consisting of rifampicin, a derivative of rifampicin, and a salt of rifampicin or the derivative.

Item 10. The preventive or therapeutic agent according to any one of items 7 to 9, wherein the preventive or therapeutic agent is used for nasal administration.

Item 11. The preventive or therapeutic agent according to any one of items 7 to 10, wherein a dose of the rifampicin compound is 3.75 mg/kg·day or less. Advantageous Effects of Invention

According to the preventive or therapeutic agent of the present invention, there is provided a medicinal agent that exhibits the effect of inhibiting aggregation of a causative protein of an HRE-related neurodegenerative disease such as ALS.

### Brief Description of Drawings

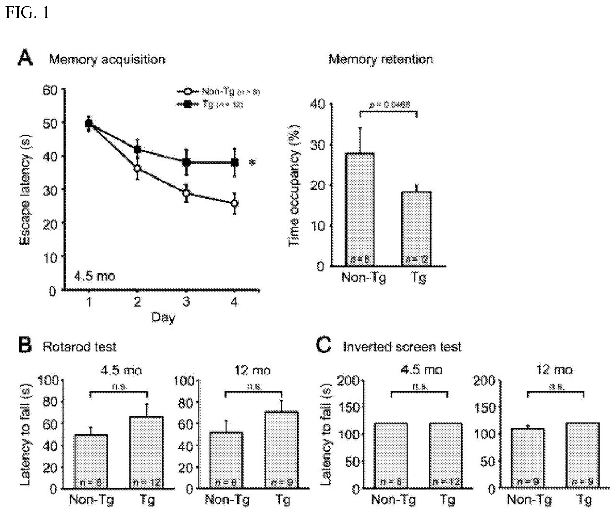

[Fig. 1] Fig. 1 shows cognitive and motor functions of C9-500 mice. Tg and non-Tg littermates were subjected to the Morris water maze (A), rotarod (B), and inverted screen tests (C) at 4.5 and 12 months (mo). Tg mice showed cognitive dysfunction in both memory acquisition and retention (probe trial) tests at 4.5 months, but had no motor dysfunction even at 12 months. * p = 0.0497 vs. non-Tg littermates. The number of mice analyzed was n = 12 (5 male and 7 female) for 4.5-month-old Tg mice, n = 8 (4 male and 4 female) for age-matched non-Tg littermates, n = 9 (7 male and 2 female) for 12-month-old Tg mice, and n = 9 (5 male and 4 female) for age-matched non-Tg littermates.

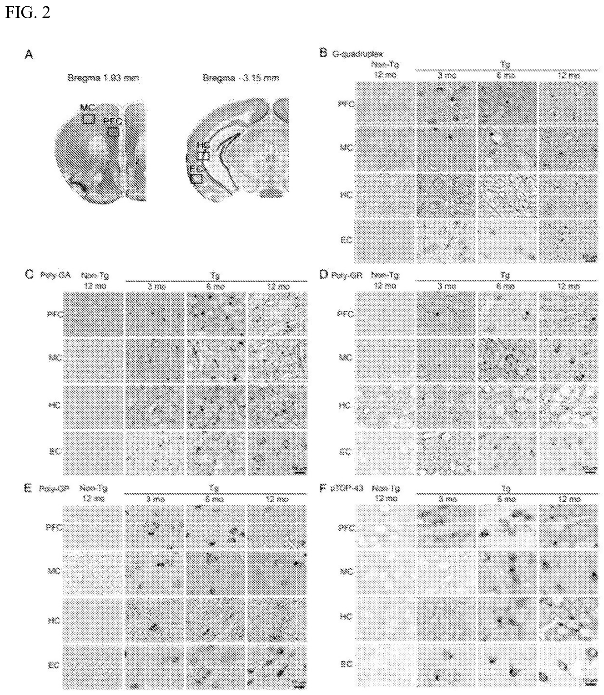

[Fig. 2] Fig. 2 shows C9orf72 HRE-related pathologies in C9-500 mice. (A) Brain sections showing the prefrontal cortex (PFC), motor cortex (MC), hippocampus (HC), and entorhinal cortex (EC) were prepared at 3, 6, and 12 months. The sections were stained for G-quadruplex (Fig. 2B), poly-GA (Fig. 2C), poly-GR (Fig. 2D), poly-GP (Fig. 2E), pTDP-43 (Fig. 2F). Accumulation of G-quadruplex and DPR in all regions detected at 3 months. Accumulation of pTDP-43 appeared in the PFC and EC at 3 months and in the MC and HC at 6 months. The number of mice analyzed was n = 3 (2 male and 1 female) for each group. These age-dependent pathologies in each brain region were observed in all mice tested.

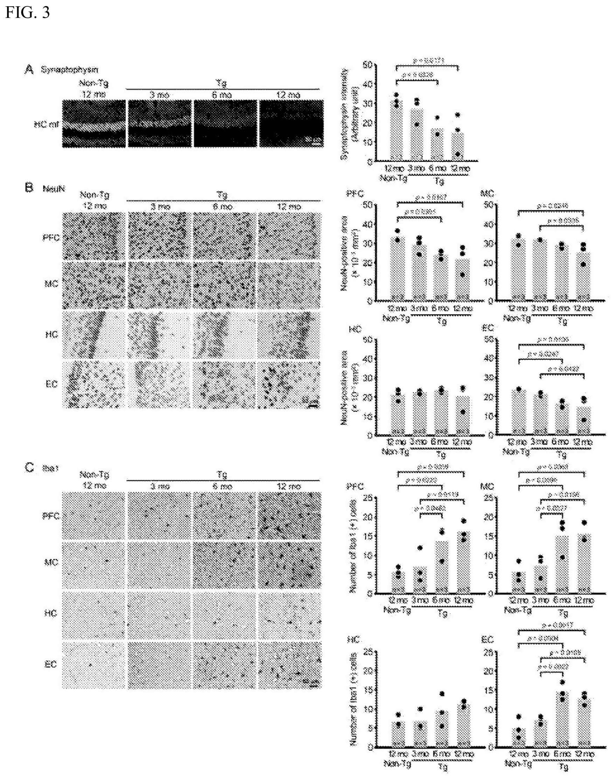

[Fig. 3] Fig. 3 shows synaptic loss, neuronal loss, and microglial activation in C9-500 mice. Brain sections were stained for synaptophysin (A), NeuN (B), Iba1 (C). The intensity of synaptophysin was measured in a constant region (50 × 50 µm) of hippocampal mossy fibers (HC mf). The levels began to decrease at 6 months. The NeuN-positive regions and Iba1-positive cells were quantified in a constant area (280 x 370 µm) of the PFC, MC, HC, and EC. Neuronal loss was detected at 6 months in the PFC and EC and at 12 months in the MC, but not in the HC. Microglial activation appeared at 6 months in the PFC, MC, and EC, but not in the HC even at 12 months. The number of mice analyzed was n = 3 (2 male and 1 female) for each group.

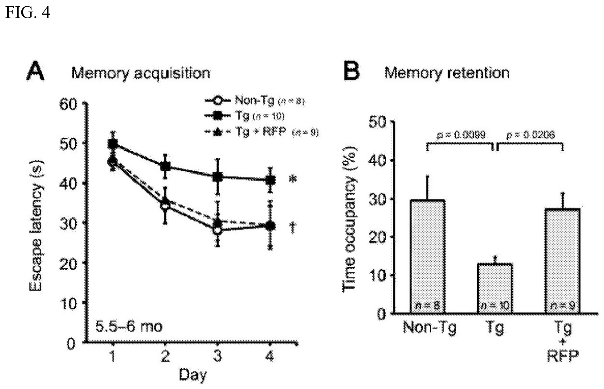

[Fig. 4] Fig. 4 shows the effect of rifampicin on the cognitive function of C9-500 mice. Nasal administration of rifampicin (RFP) at 0.1 mg/day for 1 month significantly improved cognitive function in both the memory acquisition (A) and retention (B) tests in 5.5- to 6-month-old Tg mice. * p = 0.0155 between Tg and non-Tg groups, † p = 0.0271 between Tg + RFP and Tg groups. The number of mice analyzed was n = 9 (6 male and 3 female) for rifampicin-treated Tg mice, n = 10 (6 male and 4 female) for CMC-treated Tg mice, and n = 8 (5 male and 3 female) for non-Tg littermates.

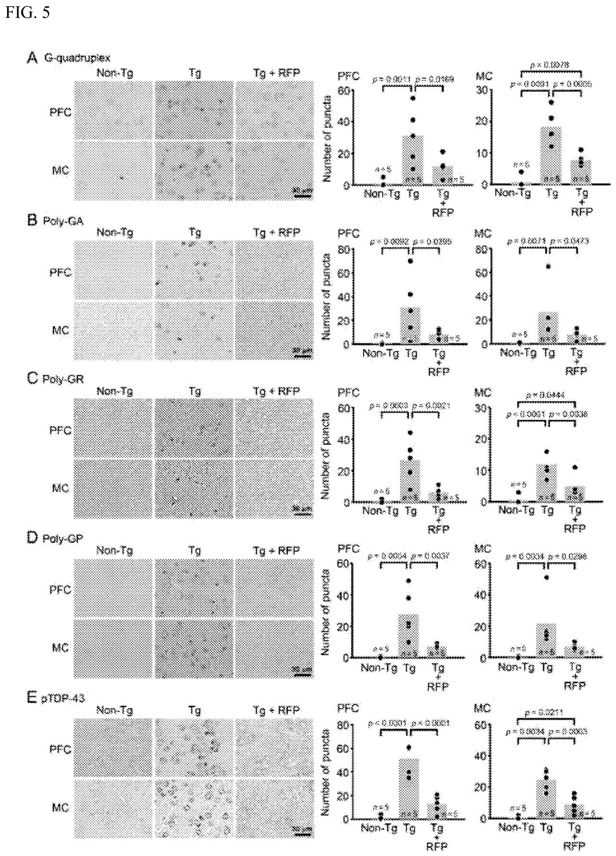

[Fig. 5] Fig. 5 shows the effects of rifampicin on HRE-related pathologies in C9-500 mice. After the behavioral test, brain sections were prepared and stained with antibodies for G-quadruplex (A), poly-GA (B), poly-GR (C), poly-GP (D), and pTDP-43 (E). These pathologies were evaluated in the PFC and MC by counting immuno-positive puncta in a certain area (280 × 370 µm). Nasal administration of rifampicin (RFP) significantly reduced the levels of G-quadruplex, DPR, and pTDP-43 puncta. The number of mice analyzed was n = 5 (3 male and 2 female) for each group.

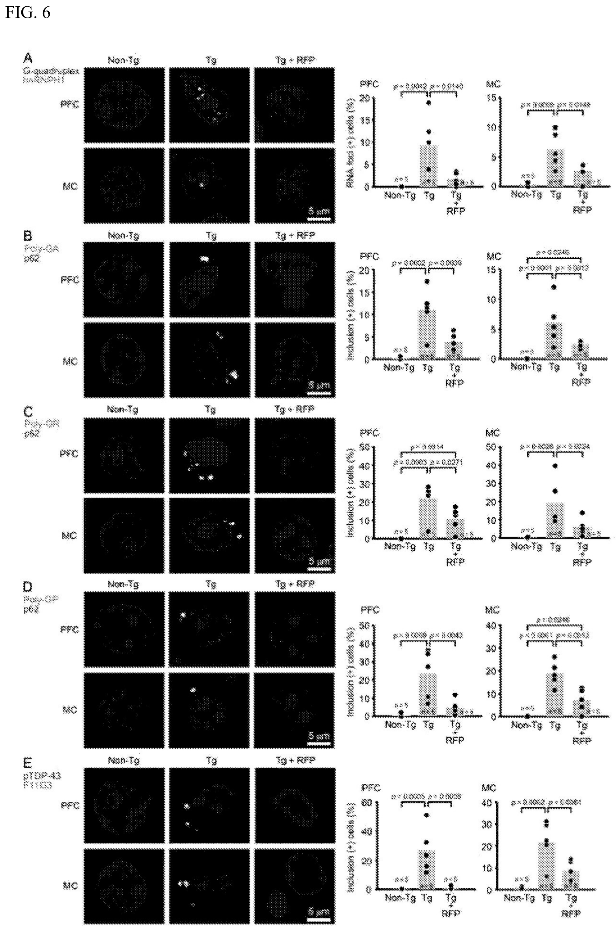

[Fig. 6] Fig. 6 shows the effects of rifampicin on RNA foci and cytoplasmic inclusions in C9-500 mice. Brain sections were stained with antibody combinations to G-quadruplex and hnRNP H1 (A) for RNA foci, DPRs and p62 for DPR inclusions (B-D), and pTDP-43 and amyloidogenic protein oligomers (F11G3) for TDP-43 oligomer inclusions (E) with a nuclear dye DAPI. These pathologies were evaluated in the PFC and MC by counting cells having double positive puncta (green + red = yellow) versus total cells (blue DAPI-positive) in a constant area (280 x 370 µm). Tg mice showed the formation of RNA foci and DPR and TDP-43 inclusions. Nasal administration of rifampicin (RFP) significantly attenuated these pathologies.

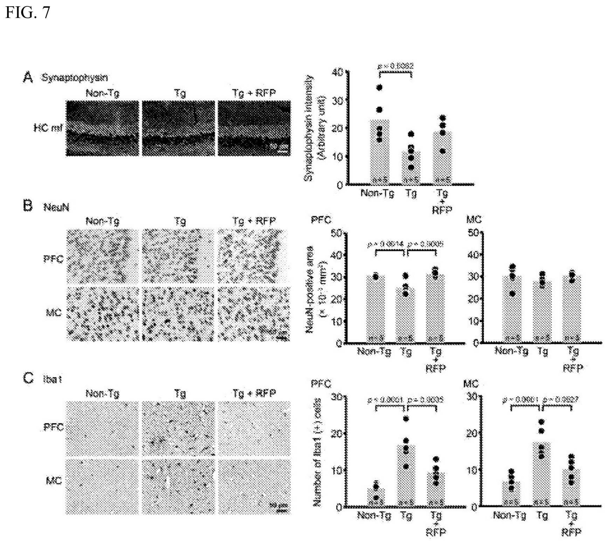

[Fig. 7] Fig. 7 shows the effects of rifampicin against synaptic loss, neuronal loss, and microglial activation in C9-500 mice. Brain sections were stained with antibodies for synaptophysin (A), NeuN (B), Iba1 (C). The intensity of synaptophysin was measured in a constant area (50 × 50 µm) of hippocampal mossy fibers (HC mf), whereas NeuN-positive areas and Iba1-positive cells were quantified in a constant area (280 × 370 µm) of the PFC and MC. Nasal administration of rifampicin (RFP) significantly restored synaptic loss in the HC, neuronal loss in the PFC, and microglial activation in the PFC and MC.

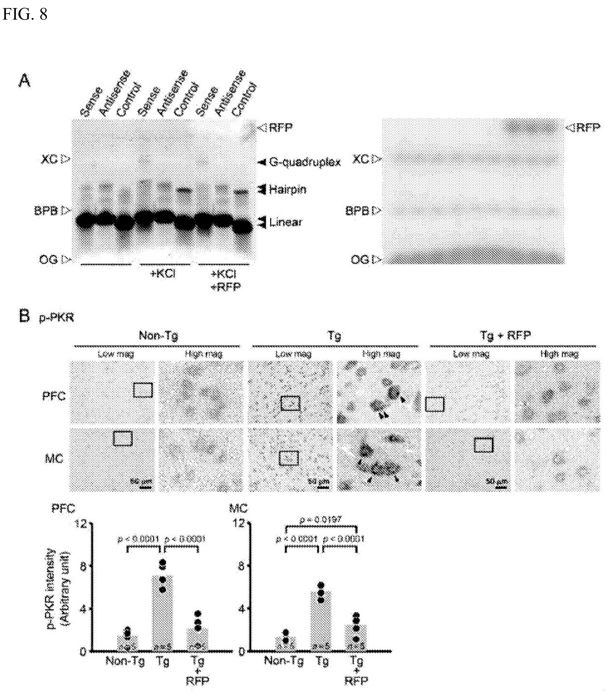

[Fig. 8] Fig. 8 shows the results of the influence of rifampicin on G-quadruplex formation and PKR phosphorylation. (A) In vitro effect of rifampicin on DNA G-quadruplex formation. Left and right photos show fluorescence and colorimetric images, respectively, of the same gel. Sense (GGGGCC)4 oligonucleotides formed G-quadruplexes in the presence of 100 mM KCl, whereas antisense (GGCCCC)4 or control (ATGC)6 oligonucleotides did not. The addition of 1 mM rifampicin (RFP) did not affect these observations. XC, xylene cyanol FF; BPB, bromophenol blue; OG, Orange G. Apparent molecular sizes of XC and BPB in 20% native gel are 45bp and 12bp, respectively. (B) Effect of rifampicin on PKR phosphorylation in C9-500 mice. Brain sections were stained with an anti-phospho-PKR (Thr446) antibody. PFC: prefrontal cortex, MC: motor cortex. The intensity of phospho-PKR was measured in a constant area (280 × 370 µm) of the PFC and MC. Compared with non-Tg littermates, Tg mice showed a marked increase in phospho-PKR level with dense punctate staining (arrowheads) in the PFC and MC. Nasal administration of rifampicin (RFP) significantly reduced the levels of phospho-PKR.

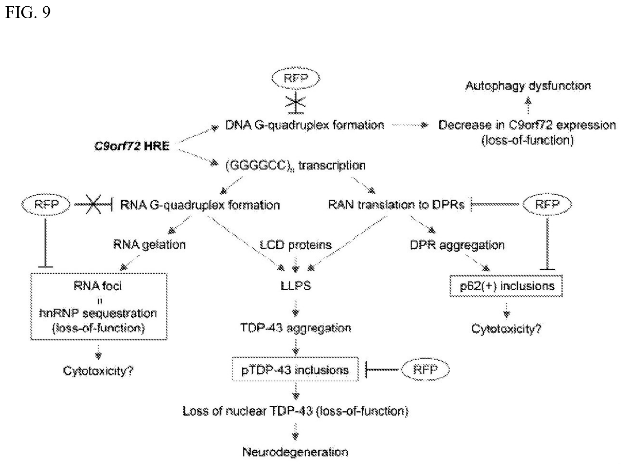

[Fig. 9] Fig. 9 shows pathways of C9orf72 HRE-induced cytotoxicity and validated actions of rifampicin. C9orf72 HRE mutation is presumed to cause disease by the loss-of-function and gain-of-toxic function of relevant proteins. HRE inhibits the transcription of C9orf72 gene by forming the G-quadruplex structure in a promoter region, which causes haploinsufficiency of C9orf72 protein and resultant autophagy dysfunction. HRE also produces aberrant sense and antisense transcripts which form the G-quadruplex and hairpin structures and sequester RNA-binding proteins into RNA foci. This sequestering may cause disturbed RNA processing, leading to the RNA-mediated cytotoxicity. The HRE-derived sense and antisense RNAs generate five DPRs by RAN translation. These DPRs self-aggregate to form inclusions that involve an autophagy- and ubiquitin-proteasome-related protein: p62. Furthermore, HRE-derived RNAs and DPRs promote cytoplasmic LLPS to form stress granules, in which RNA, RNA-binding proteins with LCD, such as TDP-43, and translation machinery are condensed. These alterations promote the formation of cytoplasmic TDP-43 inclusions and the depletion of nuclear TDP-43, which causes loss-of-function of the protein. Rifampicin (RFP) does not inhibit the G-quadruplex formation (T-shaped lines with X) but suppresses RAN translation by inhibiting PKR phosphorylation, preventing the formation of DPR and TDP-43 inclusions (T-shaped lines). Rifampicin also attenuates the formation of RNA foci by an unidentified mechanism.

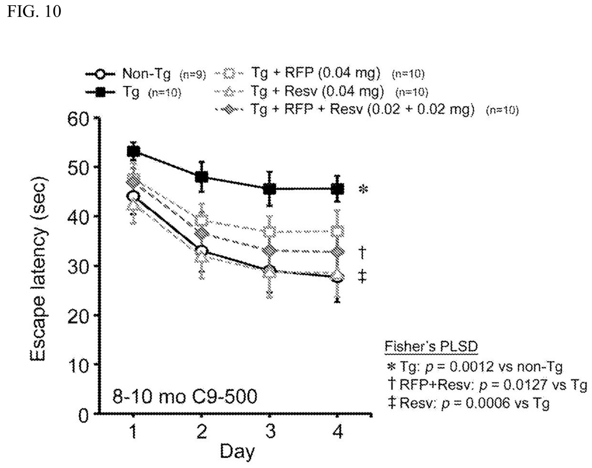

[Fig. 10] Fig. 10 shows the significant improvement of the cognitive function of FTD/ALS model mice by nasal administration of resveratrol (Resv) at 0.04 mg/day.

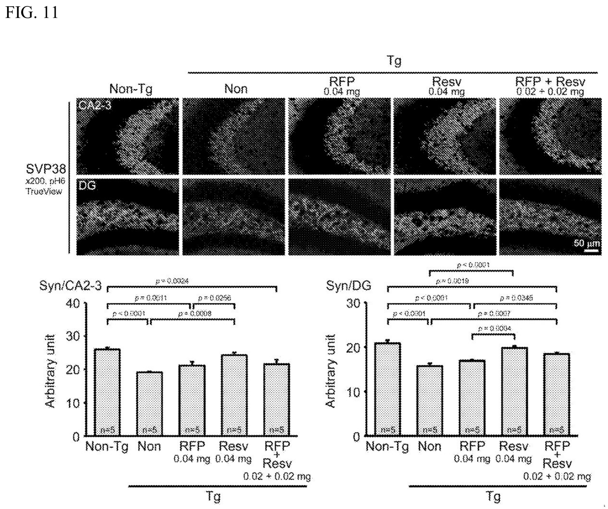

[Fig. 11] Fig. 11 shows synaptophysin levels evaluated in the hippocampus CA2-3 region and the dentate gyrus (DG). Administration of resveratrol significantly restored synaptic loss in the CA2-3 region and the DG.

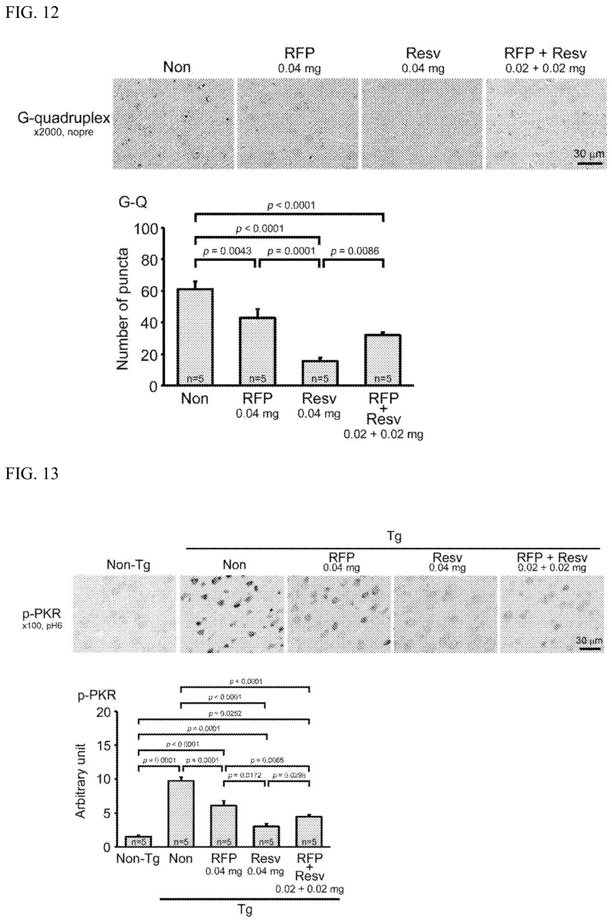

[Fig. 12] Fig. 12 shows G-quadruplex levels evaluated in the prefrontal cortex. Rifampicin, resveratrol, and a combination of rifampicin and resveratrol significantly reduced this pathology, resveratrol showed the strongest effect, and the combination showed an intermediate effect between resveratrol and rifampicin.
[Fig. 13] Fig. 13 shows phosphorylated PKR levels evaluated in the prefrontal cortex. Rifampicin, resveratrol, and a combination of rifampicin and resveratrol significantly reduced this pathology, resveratrol showed the strongest effect, and the combination showed an intermediate effect between resveratrol and rifampicin.

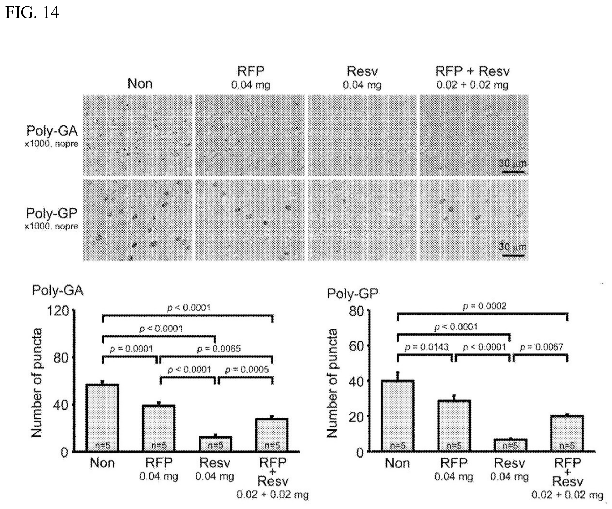

[Fig. 14] Fig. 14 shows poly-GA and poly-GP levels evaluated in the prefrontal cortex. Rifampicin, resveratrol, and a combination of rifampicin and resveratrol significantly reduced this pathology, resveratrol showed the strongest effect, and the combination showed an intermediate effect between resveratrol and rifampicin.

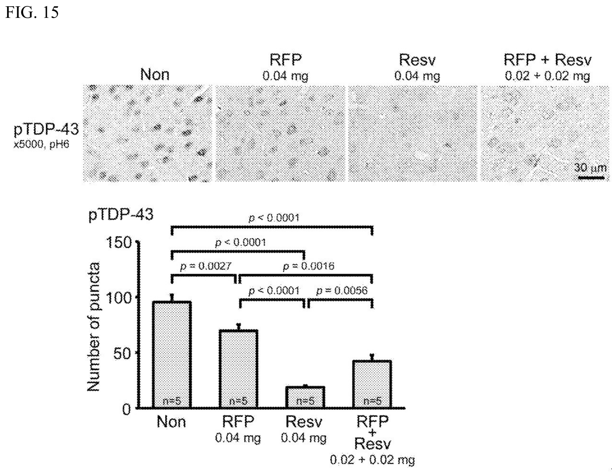

[Fig. 15] Fig. 15 shows phosphorylated TDP-43 levels evaluated in the prefrontal cortex. Rifampicin, resveratrol, and a combination of rifampicin and resveratrol significantly reduced this pathology, resveratrol showed the strongest effect, and the combination showed an intermediate effect between resveratrol and rifampicin.

### Description of Embodiments

### [Rifampicin Compound]

The pharmaceutical product of the present invention may contain, as an active ingredient, a rifampicin compound selected from the group consisting of rifampicin, a derivative of rifampicin, and a salt of rifampicin or the derivative. Rifampicin is a component known as an antibiotic.

Rifampicin is commonly a compound represented by the following Formula (I).

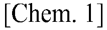

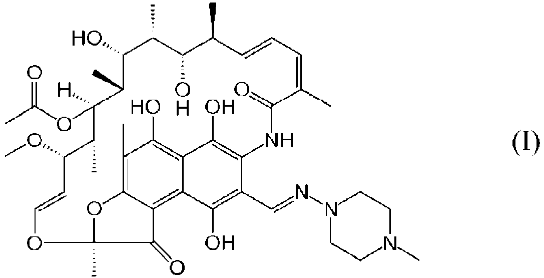

The derivative of rifampicin is not particularly limited as long as the derivative has a naphthohydroquinone or naphthoquinone structure and is pharmaceutically acceptable. Examples of the derivative include 3-Formyl-Rifamycin SV, Rifamycin S, Rifamycin B, Rifamycin SV, and a main active metabolite: 25-Desacetyl-RFP. Among the rifampicin derivatives, derivatives each having no substituent at position 3 in a 1,4-dihydroxynaphthalene structure which is involved in antibiotic activities, such as Rifamycin SV, are preferred from the viewpoint of the inhibition of the induction of a resistant bacterium resulting from long-term administration. These derivatives of rifampicin may be used singly, or two or more of them may be used in combination.

The salt of rifampicin is not particularly limited, as long as a salt of rifampicin or a rifampicin derivative can be formed and the salt is pharmaceutically acceptable. Examples of the salt include an alkali metal (e.g. potassium, sodium) salt, an alkaline earth metal (e.g. calcium, magnesium) salt, an ammonium salt, a pharmaceutically acceptable organic amine (e.g. tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine) salt, an inorganic acid salt (e.g. hydrochloride, hydrobromide, hydroiodide, sulfate, phosphate, nitrate), and an organic acid salt (e.g. acetate, lactate, tartrate, benzoate, citrate, methanesulfonate, ethanesulfonate, benzenesulphonate, toluenesulfonate, isethionate, glucuronate, gluconate). These salts may be used singly, or two or more of them may be used in combination.

As the rifampicin compound, one of rifampicin, a salt of rifampicin, a derivative of rifampicin, and a salt of the derivative of rifampicin may be selected and used, or two or more of them may be used in combination.

Among the rifampicin compounds described above, rifampicin and Rifamycin SV are preferred.

The content of the rifampicin compound in the pharmaceutical product of the present invention is not particularly limited, and is appropriately adjusted such that the rifampicin compound can be administered at the dose mentioned below. For example, the content of the rifampicin compound in the pharmaceutical product of the present invention is 0.19 w/v% or more, preferably 0.4 w/v% or more, more preferably 0.5 w/v% or more. From the viewpoint that a predetermined dose can be efficiently administered at a reduced frequency of administration, the content of the rifampicin compound in the pharmaceutical product of the present invention may be preferably 2 w/v% or more, 2.5 w/v% or more, 5 w/v% or more, or 30 w/v% or more. Furthermore, the content of the rifampicin compound in the pharmaceutical product of the present invention is 95 w/v% or less, preferably 85 w/v% or less, or 50 w/v% or less. When the pharmaceutical product of the present invention is prepared for nasal administration, the content is preferably 85 w/v% or less, or 50 w/v% or less, from the viewpoint of the achievement of the satisfactory sprayability of the nasal administration drug.

### [Resveratrol Compound]

The pharmaceutical product of the present invention may contain, as an active ingredient, a resveratrol compound selected from the group consisting of resveratrol and a derivative of resveratrol. The resveratrol compound may be combined with the rifampicin compound.

In the present invention, it is preferable to contain the resveratrol compound as an active ingredient, and it is particularly preferable to contain the resveratrol compound and not contain the rifampicin compound.

Resveratrol is 3,5,4'-trihydroxystilbene. Examples of resveratrol in the present invention include a cis-isomer of resveratrol, a trans-isomer of resveratrol, and a mixture of these isomers, preferably a trans-isomer of resveratrol. The trans-isomer of resveratrol is a compound represented by the following Formula (II).

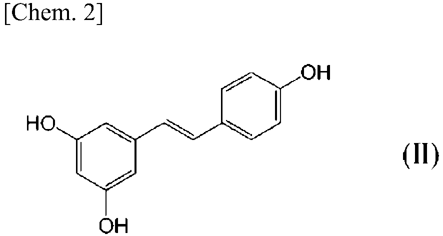

Resveratrol may be purified from a plant extract such as a lingonberry extract, a grape extract, a bilberry extract, a Japanese knotweed extract, or a gnemon tree extract, or may be produced by a chemical synthesis method, a genetic engineering method, or a microbiological method.

The derivative of resveratrol is not particularly limited as long as the derivative is pharmaceutically acceptable. Examples of the derivative include protecting groups such as an N-phenylacetyl group and a 4,4'-dimethoxytrityl (DMT) group; biopolymers such as proteins, peptides, sugars, lipids, and nucleic acids; synthetic polymers such as polystyrene, polyethylene, polyvinyl, and polyester; and a compound having a functional group, e.g. an ester group, as a derivative group. Examples of the ester group include aliphatic ester groups such as a methyl ester group and an ethyl ester group, and aromatic ester groups.

As the resveratrol compound, one of resveratrol and the resveratrol derivative may be selected and used, or two or more of them may be used in combination.

Among these resveratrol compounds, resveratrol is preferred, and a trans-isomer of resveratrol (3,5,4'-trihydroxy-trans-stilbene) is more preferred.

The content of the resveratrol compound in the pharmaceutical product of the present invention is not particularly limited, and is appropriately adjusted such that the resveratrol compound can be administered at the dose mentioned below. For example, the content of the resveratrol compound in the pharmaceutical product of the present invention is 0.19 w/v% or more, preferably 0.4 w/v% or more, more preferably 0.5 w/v% or more. From the viewpoint that a predetermined dose can be efficiently administered at a reduced frequency of administration, the content of the resveratrol compound in the pharmaceutical product of the present invention may be preferably 2 w/v% or more, 2.5 w/v% or more, 5 w/v% or more, or 30 w/v% or more. Furthermore, the content of the resveratrol compound in the pharmaceutical product of the present invention is 95 w/v% or less, preferably 85 w/v% or less, or 50 w/v% or less. When the pharmaceutical product of the present invention is prepared for nasal administration, the content is preferably 85 w/v% or less, or 50 w/v% or less, from the viewpoint of the achievement of the satisfactory sprayability of the nasal administration drug.

When the pharmaceutical product of the present invention contains a combination of the rifampicin compound and the resveratrol compound, the ratio of the rifampicin compound to the resveratrol compound is not particularly limited, and may be determined depending on the content of each of the components described above. In addition, when the pharmaceutical product of the present invention is prepared for nasal administration, the preparation may be performed such that the content of the resveratrol compound is larger than a predetermined effective amount, in consideration of the difference between the solubility of the rifampicin compound in water (for example, as for rifampicin, 2.5 mg/mL at 25°C) and the solubility of resveratrol in water (for example, as for resveratrol, 0.03 mg/mL at 25°C) and the fact that a component having lower solubility in water is more likely to be affected by the migration of a mucus layer by ciliated cells in the nasal passage and thus is more likely to flow in the digestive tract. For example, when the pharmaceutical product of the present invention is prepared for the purpose of allowing the rifampicin compound and the resveratrol compound to be absorbed at a ratio of about 1 : 1 through the paranasal cavity, the preparation can be performed such that the content of the resveratrol compound per 1 part by weight of the rifampicin compound is adjusted to an amount more than 1 part by weight. Examples of the case where the difference in solubility in water between the rifampicin compound and the resveratrol compound is taken into consideration include: a case where the pharmaceutical product of the present invention is prepared in the form of a combination drug of the rifampicin compound and the resveratrol compound; and a case where the pharmaceutical product of the present invention is prepared in the form of a kit including a medicinal agent including the rifampicin compound and a medicinal agent including the resveratrol compound, in which both of the medicinal agents are prepared using the same base containing at least water.

From these viewpoints, the lower limit of the content of the resveratrol compound per 1 part by weight of the rifampicin compound is, for example, 1/500 parts by weight or more, preferably 1/300 parts by weight or more, more preferably 1/200 parts by weight or more, still more preferably 1/100 parts by weight or more, yet still more preferably 0.05 parts by weight or more, even yet still more preferably 0.1 parts by weight or more, and particularly preferably 0.2 parts by weight or more. Preferably, the content of the resveratrol compound per 1 part by weight of rifampicin is 0.5 parts by weight or more, more preferably 0.8 parts by weight or more, still more preferably 1 part by weight or more. In addition, the content of the resveratrol compound per 1 part by weight of rifampicin may be more than 1 part by weight, such as, 1.2 parts by weight or more, 1.5 parts by weight or more, 2 parts by weight or more, 3 parts by weight or more, 4 parts by weight or more, 5 parts by weight or more, 6 parts by weight or more, 8 parts by weight or more, 10 parts by weight or more, 20 parts by weight or more, 40 parts by weight or more, 60 parts by weight or more, 80 parts by weight or more, 100 parts by weight or more, 200 parts by weight or more, or 300 parts by weight or more.

The upper limit of the content of the resveratrol compound per 1 part by weight of the rifampicin compound is, for example, 500 parts by weight or less, 300 parts by weight or less, 200 parts by weight or less, 100 parts by weight or less, 75 parts by weight or less, 50 parts by weight or less, 30 parts by weight or less, 20 parts by weight or less, 10 parts by weight or less, 5 parts by weight or less, 3 parts by weight or less, 2 parts by weight or less, 1.5 parts by weight or less, or 1.2 parts by weight or less.

### [Dosage Form]

The pharmaceutical product of the present invention is a pharmaceutical composition produced by blending the rifampicin compound and/or the resveratrol compound described above and preparing the resultant mixture into a pharmaceutical preparation by means known per se. The pharmaceutical product of the present invention may be appropriately mixed or need not be mixed with a pharmacologically acceptable base and/or an additive, depending on the administration method and/or use of the pharmaceutical product.

Examples of the pharmacologically acceptable base and/or the additive which may be or need not be contained in the pharmaceutical product of the present invention include an excipient, a thickener, a lubricant, a binder, a disintegrant, a solvent, a solubilizing agent, a suspending agent, an emulsifier, a tonicity agent, a buffering agent, a soothing agent, a stabilizer, a preserving agent (antiseptic agent), a pH adjuster, a refrigerant agent, an antioxidant, a moistening agent, an adhesive agent, and an odor-improving agent.

Examples of the excipient which may be or need not be contained in the pharmaceutical product of the present invention include lactose, white sugar, D-mannitol, starch, corn starch, crystalline cellulose, and light anhydrous silicic acid. Examples of the thickener include polyhydric alcohols such as glycerin and macrogol; celluloses such as methyl cellulose, carboxymethylcellulose, and hydroxypropylmethyl cellulose; hydrophilic polymers such as polyvinyl alcohol, polyvinylpyrrolidone, carboxyvinyl polymer, sodium carboxymethylcellulose, methyl cellulose, hydroxymethyl cellulose, hydroxyethyl cellulose, and hydroxypropyl cellulose (preferably sodium carboxymethylcellulose); sodium alginate; chondroitin sulfate; cyclodextrin; d-α-tocopherol polyethylene glycol 1000 succinate; and polyethylene glycol. Examples of the lubricant include magnesium stearate, calcium stearate, talc, and colloidal silica. Examples of the binder include crystalline cellulose, white sugar, D-mannitol, dextrin, hydroxypropyl cellulose, hydroxypropylmethyl cellulose, polyvinylpyrrolidone, starch, sucrose, gelatin, methyl cellulose, and sodium carboxymethylcellulose. Examples of the disintegrant include starch, carboxymethylcellulose, calcium carboxymethylcellulose, croscarmellose sodium, sodium carboxymethyl starch, and L-hydroxypropyl cellulose. Examples of the solvent include water, ethanol, isopropyl alcohol, acetone, propylene glycol, macrogol, sesame oil, and corn oil, and it is preferred for the solvent to contain at least water. Examples of the solubilizing agent include celluloses such as methyl cellulose, carboxymethylcellulose, and hydroxypropylmethyl cellulose; polyethylene glycol, propylene glycol, D-mannitol; and benzyl benzoate, ethanol, tris-aminomethane, cholesterol, triethanolamine, sodium carbonate, sodium citrate, polyvinylpyrrolidone, and macrogol. Examples of the suspending agent include surfactants such as stearyl triethanolamine, sodium lauryl sulfate, lauryl aminopropionic acid, lecithin, benzalkonium chloride, benzethonium chloride, glycerin monostearate, polyoxyethylene hydrogenated castor oil, and polysorbate; polyhydric alcohols such as glycerin and macrogol; sugars such as sorbitol, mannitol, and sucrose; celluloses such as methyl cellulose, carboxymethylcellulose, and hydroxypropylmethyl cellulose; hydrophilic polymers such as polyvinyl alcohol, polyvinylpyrrolidone, carboxyvinyl polymer, sodium carboxymethylcellulose, methyl cellulose, hydroxymethyl cellulose, hydroxyethyl cellulose, and hydroxypropyl cellulose; and chondroitin sulfate. Examples of the tonicity agent include glucose, D-sorbitol, sodium chloride, glycerin, D-mannitol, potassium chloride, concentrated glycerin, propylene glycol, and sucrose. Examples of the buffering agent include phosphate (e.g. sodium hydrogenphosphate, sodium dihydrogen phosphate), boric acid, borax, acetate (e.g. sodium acetate), carbonate (e.g. sodium carbonate, calcium carbonate, potassium carbonate), citric acid, and sodium L-glutamate. Examples of the soothing agent include benzyl alcohol, chlorobutanol, propylene glycol, ethyl aminobenzoate, and lidocaine. Examples of the stabilizer include sulfur compounds such as sodium sulfite, sodium bisulfite, sodium metabisulfite, sodium thiosulfate, Rongalite, thioglycerol, thioglycolic acid, thiolactic acid, cysteine, glutathione, thioacetic acid, methionine, thiosorbitol, thioglucose and thiourea; inorganic acids and salts thereof such as boric acid, borax, phosphoric acid, metaphosphoric acid, sodium carbonate and sodium hydrogen carbonate; organic acids such as formic acid, oxalic acid, tartaric acid, citric acid and edetic acid, and salts thereof (e.g. sodium edetate); acid amides such as acetamide, diethylacetamide, nicotinamide, urea and barbital; urea derivatives; polyhydric alcohols such as glycol, propylene glycol, glycerin, polyethylene glycol, glucose and ascorbic acid; sugars; phenols such as phenol, thymol, quinone, coumarone and isocoumarone; dibutylhydroxytoluene; amino acids such as glycine, glutamic acid, lysine, phenylalanine, casein and edestin; and proteins. Examples of the emulsifier include glycerin ester (e.g. glyceryl monooleate), saponin (e.g. sophora saponin, quillaia extract, soybean saponin), sucrose fatty acid ester, lecithin (e.g. vegetable lecithin, egg yolk lecithin, soybean lecithin), polyhydric alcohol (e.g. oleyl alcohol, stearyl alcohol, cetyl alcohol), fatty acid ester (e.g. octyldodecyl myristate), medium-chain triglyceride (MCT), various surfactants (e.g. an alkyl benzene sulfonate-type emulsifier, benzalkonium chloride, sorbitan sesquioleate, dodecylbenzenesulfonic acid), and triethanolamine. Examples of the preserving agent (antiseptic agent) include p-hydroxybenzoates such as propyl-p-hydroxybenzoate and butyl-p-hydroxybenzoate; parabens such as methylparaben, ethylparaben, propylparaben and butylparaben; invert soaps such as benzalkonium chloride, benzethonium chloride, chlorhexidine gluconate and cetylpyridinium chloride; alcohol derivatives such as chlorobutanol, benzyl alcohol and phenethyl alcohol; organic acids and salts thereof, such as sodium dehydroacetate, sorbic acid and sodium sorbate; and phenols such as p-chloromethoxyphenol and p-chlorometacresol. Examples of the pH adjuster include sodium hydroxide, potassium hydroxide, trisodium phosphate, disodium hydrogenphosphate, hydrochloric acid, nitric acid, citric acid, boric acid, and acetic acid. Examples of the refrigerant agent include 1-menthol, camphor, and peppermint water. Examples of the antioxidant include sulfite, ascorbic acid, citric acid, and sodium edetate. Examples of the moistening agent include propylene glycol, polysorbate, macrogol, and glycerin. Examples of the adhesive agent include hydroxypropyl cellulose, hydroxypropylmethyl cellulose 2208, carboxyvinyl polymer, propylene glycol, and polysorbate 80. Examples of the odor-improving agent include trehalose, malic acid, maltose, potassium gluconate, anise essential oil, vanilla essential oil, cardamom essential oil, and a crude drug ingredient.

The pharmaceutical product of the present invention may be in the form of a liquid preparation or a solid preparation, and is preferably in the form of a liquid preparation. When it is intended to prepare a liquid preparation, the liquid preparation can be produced by mixing the rifampicin compound and/or the resveratrol compound and optionally a solvent, a solubilizing agent, a suspending agent, a tonicity agent, a buffering agent, a soothing agent, and the like, and dissolving, suspending or emulsifying these components. When the pharmaceutical product of the present invention is prepared as a nasal administration drug, it is also preferable to further add a thickener to increase the viscosity of the solution, thereby imparting retentivity. When it is intended to prepare a solid preparation, the solid preparation can be produced by mixing the rifampicin compound and/or the resveratrol compound and optionally an excipient, a binder, a disintegrant or any other appropriate additive together homogeneously, then granulating the resultant mixture by an appropriate granulation method to produce granules, and then making the granules into a powder or fine granules by an appropriate drying method.

When the pharmaceutical product of the present invention is prepared as a nasal administration drug, the pharmaceutical product may be packed in a container for nasal administration upon use. As the container for nasal administration, a commercially available container may be used as appropriate.

A more specific example of an aspect when the pharmaceutical product of the present invention contains a combination of the rifampicin compound and the resveratrol compound is a combination drug of the rifampicin compound and the resveratrol compound. The combination drug is a pharmaceutical composition in which the rifampicin compound and the resveratrol compound are contained in a mixed state. According to this combination drug, the rifampicin compound and the resveratrol compound can be administered simultaneously in any dosage form. Another specific example of the aspect of the pharmaceutical product of the present invention in this case is a kit including a medicinal agent containing the rifampicin compound and a medicinal agent containing the resveratrol compound. These medicinal agents may be prepared using the same base and/or the same additive, or may be prepared using bases and/or additives which are respectively selected for the rifampicin compound and the resveratrol compound. According to the kit, the rifampicin compound and the resveratrol compound can be separately administered in any dosage form. In addition, when the kit is applied to a nasal administration device equipped with a single cartridge packed with the medicinal agent containing the rifampicin compound and another single cartridge packed with the medicinal agent containing the resveratrol compound per nasal administration device, the kit enables the simultaneous administration of both of the components.

### [Dose and Usage]

The pharmaceutical product of the present invention may be prepared in a form suitable for any one of oral administration, subcutaneous administration, and nasal administration. Among administration routes, the pharmaceutical product of the present invention is more preferably prepared in a form suitable for nasal administration, from the viewpoint that the administration route is non-invasive and a more preferable side-effect-suppressing effect can be achieved and from the viewpoint that the administration is performed at a smaller dose and/or for a longer period.

With regard to the dose of the pharmaceutical product of the present invention to humans, the lower limit of the dose of the rifampicin compound is, for example, 0.15 mg/kg·day or more, preferably 0.3 mg/kg·day or more, more preferably 0.75 mg/kg·day or more, still more preferably 1 mg/kg·day or more, from the viewpoint that the pharmacological effect can be exerted. In addition, the upper limit of the dose of the rifampicin compound to humans is, for example, 3.75 mg/kg·day or less, preferably 2.5 mg/kg/day or less, more preferably 2 mg/kg·day or less, still more preferably 1 mg/kg·day or less, yet still more preferably 0.5 mg/kg·day or less, even yet still more preferably 0.4 mg/kg·day or less, from the viewpoint that the side effects can be reduced.

When the pharmaceutical product of the present invention contains a combination of the rifampicin compound and the resveratrol compound, a lower dose is also acceptable as the dose of the rifampicin compound to humans. The lower limit of the dose of the rifampicin compound to humans in this case is, for example, 0.001 mg/kg·day or more, preferably 0.002 mg/kg·day or more, more preferably 0.003 mg/kg·day or more, still more preferably 0.005 mg/kg·day or more, still more preferably 0.01 mg/kg·day or more, from the viewpoint that the pharmacological effect can be exerted. From the viewpoint that a more preferable pharmacological effect can be exerted, the lower limit of the dose of the rifampicin compound to humans is, for example, 0.025 mg/kg·day or more, preferably 0.05 mg/kg·day or more, more preferably 0.1 mg/kg·day or more. Furthermore, the upper limit of the dose of the rifampicin compound to humans is 1.5 mg/kg·day or less, preferably 1 mg/kg·day or less, more preferably 0.5 mg/kg·day or less, still more preferably 0.1 mg/kg·day or less, yet still more preferably 0.07 mg/kg·day or less, from the viewpoint that the side effects can be further reduced.

With respect to the dose of the pharmaceutical product of the present invention to humans, the dose of the resveratrol compound (when combined with the rifampicin compound and when not combined with the rifampicin compound) is, for example, 0.001 mg/kg·day or more, preferably 0.002 mg/kg·day or more, more preferably 0.003 mg/kg·day or more, still more preferably 0.005 mg/kg·day or more, yet still more preferably 0.01 mg/kg·day or more, 0.05 mg/kg·day or more, or 0.1 mg/kg·day or more, from the viewpoint that the pharmacological effect can be exerted. The upper limit of the dose of the resveratrol compound to humans is not particularly limited and is, for example, 3.75 mg/kg·day or less, preferably 2.5 mg/kg·day or less, more preferably 2 mg/kg·day or less, still more preferably 1.5 mg/kg·day or less or 1 mg/kg·day or less, still more preferably 0.5 mg/kg·day or less, yet still more preferably 0.4 mg/kg·day or less, 0.3 mg/kg·day or less, 0.2 mg/kg·day or less, or 0.15 mg/kg·day or less, particularly preferably 0.1 mg/kg·day or less, most preferably 0.07 mg/kg·day or less.

The pharmaceutical product of the present invention is suitable for continuous administration because it can be administered at a small dose. The period for administration of the pharmaceutical product of the present invention to humans is, for example, 1 month or longer, preferably 3 months or longer. The pharmaceutical product of the present invention can be administered for an even longer period. Thus, more preferred examples of the period for administration to humans include, for example, 6 months or longer, preferably 1 year or longer, more preferably 1.5 years or longer, still more preferably 2 years or longer, yet still more preferably 2.5 years or longer. The period for administration may be 3 years or longer. The upper limit of the period for administration to humans is not particularly limited, and is, for example, 10 years or shorter, 8 years or shorter, 6 years or shorter, or 4 years or shorter. The upper limit of the period for administration may be 3 years or shorter. The administration interval is every day, every other day, or once or twice per week, preferably every other day or every day, still more preferably every day.

### [Subject to Be Administered]

As the first use, the pharmaceutical product of the present invention can be used for the prevention or treatment of a neurodegenerative disease (TDP-43 proteinopathy) caused by accumulation of TDP-43. The neurodegenerative disease caused by the accumulation of TDP-43 includes familial and sporadic neurodegenerative diseases. Specific examples of the diseases include amyotrophic lateral sclerosis (ALS) of TDP-43 proteinopathy and frontotemporal dementia (FTD) of TDP-43 proteinopathy.

In addition, the pharmaceutical product of the present invention can be used for the prevention or treatment of amyotrophic lateral sclerosis (ALS) as the second use. Amyotrophic lateral sclerosis (ALS) includes TDP-43 proteinopathy, FUS proteinopathy, and SOD 1 type. In particular, a form of the pharmaceutical product of the present invention in which the resveratrol compound is contained as an active ingredient (most preferably, a form in which the resveratrol compound is contained as an active ingredient, and the rifampicin compound is not contained as an active ingredient) is preferably used for the prevention or treatment of ALS of TDP-43 proteinopathy.

Examples of the first use and the second use include neurodegenerative diseases caused by C9orf72 gene abnormality and/or TDP-43 gene abnormality, and a preferred example includes a neurodegenerative disease caused by TDP-43 gene abnormality.

### Examples

Hereinafter, the present invention will be described more specifically with reference to Examples, but the present invention is not limited thereto.

### [Test Example 1]

### 1. Materials and Methods

### 1.1 Mice

FVB/NJ-Tg(C9orf72)500Lpwr/J mice were purchased from The Jackson Laboratory (Bar Harbor, ME, USA). The mouse strain was generated as a model for the C9orf72-linked FTD/ALS expressing the human C9orf72 gene with 500 hexanucleotide (GGGGCC) repeats (hereinafter, it is also described as "FTD/ALS model mice") using a bacterial artificial chromosome (BAC) vector. Transgenic (Tg) mice were crossed with wild-type FVB/NJc1 mice, and the transgene was maintained as a heterozygote at the animal facility in our company.

### 1.2. Behavioral Test

The cognitive function of the mice was assessed in a Morris water maze test at the age of 4.5 months, and the motor function of the mice was assessed in the rotarod and inverted screen tests at the age of 4.5 months and at the age of 12 months in an above-described manner.

### 1.3. Immunohistochemical Analysis

To study age-dependent neuropathology in mice, brain sections of Tg and non-Tg littermates were prepared at the age of 3 months, at the age of 6 months, and at the age of 12 months as described above. For staining of pTDP-43, synaptophysin, and NeuN, the sections were boiled in 10 mM of citrate buffer solution (pH6) for 30 min to expose antigens. The sections were blocked overnight with 10% calf serum, and then the resultant sections were stained with antibodies for DNA/RNA G-quadruplex (BG4; Absolute Antibody, Cleveland, UK), poly-GA, poly-GP, poly-GR (all manufactured by Cosmo Bio Co., Ltd., Tokyo, Japan), pSer409/410-TDP-43 (Cosmo Bio Co., Ltd.), synaptophysin (SVP-38; Sigma-Aldrich, St. Luis, MO, USA), NeuN (Chemicon International, Inc., Temecula, CA, USA), and Iba1 (FUJIFILM Wako Pure Chemical Corporation, Osaka, Japan). After staining, a biotin-labeled secondary antibody (Vector Laboratories, Burlingame, CA, USA), a horseradish peroxidase (HRP)-labeled avidin biotin complex (Vector Laboratories), and HRP substrate diaminobenzidine (DAB), or an FITC-labeled secondary antibody (The Jackson Laboratory) was used only for synaptophysin. The stained specimens were observed with a fluorescence microscope BZ-X800 (KEYENCE CORPORATION, Osaka, Japan), and images of specific brain regions were photographed. Neuropathology was evaluated using NIH ImageJ software (ImageJ bundled with 64 bit Java 1.8.0_172; https://imagej.nih.gov/ij/, accessed on 27 March 2022) by measuring the staining intensity or area of each of the photographs or counting positive puncta or cells in a certain area.

To examine the formation of nuclear and cytoplasmic inclusions, the sections were double stained with antibodies to G-quadruplex and hnRNP H1 (Proteintech, Rosemont, IL, USA) without pretreatment for RNA foci, and pSer409/410-TDP-43 and amyloidogenic protein oligomers (F11G3; Sigma-Aldrich) after pretreatment at pH6 for TDP-43 oligomer inclusions. For DPR inclusions, the sections were double-stained with antibodies to poly-GA, poly-GP, poly-GR, and SQSTM1/p62 (A-6; Santa Cruz Biotechnology, Dallas, TX, USA) without pretreatment. After staining, FITC- and rhodamine-labeled secondary antibodies (Jackson Laboratory) were used. The specimens were then treated with TrueBlack Plus Lipofuscin (Biotium, Fremont, CA, USA) to quench the autofluorescence and mounted using a Vectashield vibrance antifade mounting medium with DAPI (Vector Laboratories). Images of the stained sections were taken with a BZ-X800 fluorescence microscope, and cells having inclusion bodies were counted in a certain area.

### 1.4. Rifampicin Treatment

4-and-a half to 5-month-old FTD/ALS model mice were divided into two groups. One group received rifampicin and the other group received carboxymethylcellulose (CMC) every day from Monday to Friday for 4 weeks. Rifampicin (RFP; Sigma-Aldrich, Rifampicin ≥ 97% (HPLC), powder, a.k.a.: 3-(4-Methylpiperazinyliminomethyl)rifamycin SV, Rifamycin AMP, Rifampin, R3501)) was dissolved to 10 mg/mL in 0.5% low-viscosity CMC (Sigma-Aldrich). 10 µL of rifampicin (i.e. 0.1 mg) or CMC solution was administered nasally. Non-Tg littermates were treated with CMC alone. After the 1-month administration, cognitive function of the mice was tested by the water maze test, during which the rifampicin administration continued. After the behavioral test, each group was divided into two groups: one group for immunohistochemical analysis and the other group for future biochemical analysis. For immunohistochemical analysis, brain sections were prepared as described above, while for biochemical analysis, whole brains were removed and frozen at -80°C until use.

### 1.5. Gel Shift Assay for G-Quadruplex

The gel shift assay to detect G-quadruplex formation was performed. Sense (GGGGCC)₄, antisense (GGCCCC)₄, and Control (ATGC)₆ oligonucleotides were synthesized and solubilized to 100 µM in 0.89 M of Tris-borate buffer, pH8.3 containing 0.02 M of EDTA (TBE). KCl was solubilized to 400 mM in TBE, and rifampicin was solubilized to 100 mM in DMSO. 5 µL of DNA solution, 25 µL of KCL or TBE alone, and 1 µL of rifampicin or DMSO alone were mixed with 69 µL of TBE to make the final concentrations 5 µM of DNA, 100 mM of KCl, and 1 mM of rifampicin. The mixtures were incubated at 98°C for 3 min and slowly cooled to room temperature. The samples were mixed with Blue/Orange Loading Dye (Promega Corporation, Madison, WI, USA) and subjected to native PAGE with 20% polyacrylamide gel (Novex TBE Gels, Invitrogen, Carlsbad, CA, USA) and TBE running buffer containing 50 mM KCl. The gels were stained with SYBR Gold nucleic acid gel stain (Invitrogen) for 10 min, and images were acquired using an ImageQuant LAS 500 image analyzer (GE Healthcare, Hino, Japan).

### 1.6. Immunohistochemical Studies of Phosphorylated PKR

To validate the possibility that rifampicin affects RAN translation, the levels of phosphorylated PKR and a regulator of RAN translation were examined immunohistochemically. Brain sections were boiled in 10 mM of citrate buffer solution (pH6) for 30 min to expose the antigens. After blocking, the sections were stained with an anti-phospho-PKR (Thr446) antibody (MilliporeSigma, Burlington, MA, USA), followed by a biotin-labeled secondary antibody, HRP-labeled avidin biotin complex, DAB. The stained specimens were observed with a BZ-X800 fluorescence microscope, and the levels of phosphorylated PKR were measured by quantifying the staining intensity in a constant area using NIH ImageJ software.

### 1.7. Statistical Analysis

All experiments and data analyses were performed under unblinded conditions. Upon comparison of average values among two groups or more, ANOVA or two-factor repeated measures ANOVA (for the Morris water maze test) was performed using Fisher's PLSD test. Differences with a p-value < 0.05 were considered significant.

### 2. Results

First, the pathological phenotypes of FTD/ALS model mice were examined. The cognitive function of the mice was assessed in a Morris water maze test at the age of 4.5 months, and the motor function of the mice was assessed in the rotarod and inverted screen tests at the age of 4.5 months and at the age of 12 months. Compared with non-Tg littermates, Tg mice showed significantly impaired memory performance at 4.5 months, but no motor function deficiency (Fig. 1). The motor function of Tg mice was normal even at 12 months. These mice are considered to be a slowly progressive type.

Next, the neuropathology of the mice was studied by immunohistochemistry. Brain sections were generated at 3, 6, and 12 months and stained with antibodies for DNA/RNA G-quadruplex, poly-GA, poly-GR, poly-GP, and pTDP-43. Vulnerable sites in FTD and ALS: prefrontal cortex (PFC), motor cortex (MC), hippocampus (HC), and entorhinal cortex (EC) were examined (Fig. 2A). G-quadruplex appeared at 3 months in all tested brain regions (Fig. 2B). The three DPRs also started to accumulate at 3 months in all brain regions (Fig. 2C to 2E). pTDP-43 was detected at 3 months in the PFC and EC and at 6 months in the MC and HC (Fig. 2F).

Then, the brain sections were stained with antibodies for synaptophysin, the neuronal marker NeuN, and the microglial marker Iba1. Synaptophysin levels were measured in the hippocampal mossy fibers. Significant synaptic loss was detected in Tg mice at 6 months (Fig. 3A). NeuN-positive regions and Iba1-positive cells were quantified in the PFC, MC, HC, and EC. Significant neuronal loss appeared in the PFC and EC at 6 months and in the MC at 12 months, but barely in the HC even at 12 months (Fig. 3B). In addition, significant microglial activation was observed at 6 months in the PFC, MC, EC, as was a similar but not significant trend in the HC (Fig. 3C). These results indicate that G-quadruplex formation and DPR accumulation were the initial pathology appearing in FTD/ALS model mice followed by pTDP-43 accumulation. Furthermore, neurodegeneration was associated with the accumulation of pTDP-43, and the most susceptible brain sites in FTD/ALS model mice were the PFC and EC.

4.5- to 5-month-old mice were used to examine the therapeutic potential of rifampicin against C9orf72-linked FTD. Tg mice were divided into two groups: one group received nasal rifampicin at 0.1 mg/day for 1 month and the other group received CMC alone. Non-Tg littermates were treated with CMC. After treatment, the cognitive function of the mice was evaluated by the water maze test. Nasal administration of rifampicin significantly improved the memory performance of Tg mice to a level similar to non-Tg littermates (Fig. 4).

After completion of the behavioral test, each group was divided into two groups: one for immunohistochemical analysis and the other for future biochemical analysis. In the immunohistochemistry, brain sections were prepared and stained with antibodies for G-quadruplex, poly-GA, poly-GR, poly-GP, and pTDP-43. These pathologies were evaluated in the PFC and MC. Nasal administration of rifampicin significantly reduced the levels of G-quadruplex, DPR, and pTDP-43 puncta (Fig. 5).

Further, it was examined whether these pathological molecules constituted RNA foci and cytoplasmic inclusions. RNA foci are formed dominantly in the nucleus and occasionally in the cytoplasm and composed of RNA G-quadruplex and RNA-binding proteins, such as hnRNP H. DPR inclusions are commonly formed in the cytoplasm and occasionally in the nucleus, and involve autophagy- and ubiquitin-proteasome-related p62 protein. TDP-43 inclusions are formed in the cytoplasm, and are positive for aggregated pTDP-43.

Thus, brain sections were stained with antibody combinations to G-quadruplex and hnRNP H for RNA foci, DPRs and p62 for DPR inclusions, and pTDP-43 and amyloidogenic protein oligomers (F11G3) for TDP-43 oligomer inclusions. Tg mice showed the formation of RNA foci and DPR and TDP-43 cytoplasmic inclusions in the PFC and MC (Fig. 6). Nasal administration of rifampicin significantly attenuated these pathologies.

Then, brain sections were stained with antibodies for synaptophysin, NeuN, and Iba1. Nasal administration of rifampicin significantly suppressed synaptic loss in hippocampal mossy fibers, neuronal loss in the PFC, and microglial activation in the PFC and MC (Fig. 7). These results suggest a therapeutic potential of nasal administration of rifampicin in the prevention of C9orf72-linked diseases.

Next, the mechanisms by which rifampicin attenuated C9orf72 HRE-related pathologies were examined. The present inventors speculated that rifampicin may interact with DNA/RNA to inhibit G-quadruplex formation. To validate this hypothesis, the effect of rifampicin in vitro on G-quadruplex formation by synthetic (GGGGCC)₄ oligonucleotides was examined. A gel shift assay revealed that the DNA formed G-quadruplex in the presence of 100 mM of KCl and that the addition of 1 mM of rifampicin failed to inhibit this formation (Fig. 8A). Then, the present inventors considered that rifampicin may suppress RAN translation by inhibiting PKR phosphorylation like metformin. The levels of phosphorylated PKR in mouse brains were examined by immunohistochemistry. Compared with non-Tg littermates, Tg mice showed a marked increase in phosphorylated PKR level in the PFC and MC (Fig. 8B). Nasal administration of rifampicin significantly attenuated the levels of phosphorylated PKR. These results suggest that rifampicin prevents brain pathologies by suppressing RAN translation via inhibition of PKR phosphorylation.

### 3. Discussion

The FTD/ALS model mice used in this example were originally created as a model of C9orf72-linked FTD/ALS. According to the founder, the mice are divided into two subsets: an acute, rapidly progressive type and a slowly progressive type. The acute progressive type mice (which corresponds to about 30 to 35% of all female mice) developed extensive neuronal loss in layers II/III throughout the cerebral cortex, layer V of the MC, the HC, cerebellum, and spinal cord at 2 to 5 months and showed hindlimb gait abnormalities at 4 months and a dramatic decrease in survival rate between 5 to 20 months. Meanwhile, the slowly progressive mice showed a loss of motor neurons in the spinal cord, focal neurodegeneration in the neocortex, milder degeneration in the cerebellum, and no degeneration in the HC at 18 months. Male mice did not show a decrease in survival rate even at 1 year, but a large percentage of them (~43 to 45%) developed phenotypes similar to those found in slowly progressive female mice by 1 year. Sense and antisense RNA foci were detected in the MC, HC, cerebellum, and spinal cord as early as 2 months. Poly-GA aggregates were detected throughout the brain at 2 months, with the first appearance in the occipital cortex, and increased with age and disease. Poly-GP aggregates were also detected in the neocortex and thalamus in acute end-stage mice. Finally, aggregates of TDP-43 were detected in degenerating neurons throughout the brain, including the HC and layers II/III and V of the MC, in acute end-stage mice.

In this example, the pathological phenotypes of FTD/ALS model mice purchased from The Jackson Laboratory were first determined and bred at the present inventor's animal facility. As a result, the mice showed cognitive dysfunction at 4.5 months, but no motor dysfunction even at 12 months. It was observed that apparent neuropathologies, including RNA foci, DPR and TDP-43 inclusions, synaptic loss, neuronal loss, and microglial activation, in the PFC, MC, HC, and EC regions in an age-dependent manner starting from 3 months.

Neurodegenerative diseases are believed to be generally caused by the gain-of-toxic function of amyloidogenic proteins. These proteins have been shown to become neurotoxic when they aggregate into oligomers For example, AD is initiated by the formation of Aβ oligomers and is progressed by toxic aggregates of tau proteins. FTD is elicited by the oligomerization of tau or TDP-43, and TDP-43 inclusions are characteristics in most cases of ALS. DLB and Parkinson's disease are associated with α-synuclein oligomers. Meanwhile, C9orf72 HRE mutations have been suggested to cause both gain-of-toxic function and loss-of-function. C9orf72 HRE produces RNA foci and DPR and TDP-43 inclusions, all of which have cytotoxic actions. At the same time, this mutation negatively affects the expression of C9orf72 by forming G-quadruplex structures in a promoter region, leading to haploinsufficiency of the protein. Furthermore, RNA foci may sequester some RNA-binding proteins, which may also result in loss-of-function of these proteins. In addition, HRE-derived RNAs and DPRs promote cytoplasmic LLPS and stress granule formation, in which TDP-43 tends to self-aggregate to form cytoplasmic inclusions. This is believed to cause the depletion of nuclear TDP-43, leading to loss-of-function of the protein.

As described above, the present inventors showed that nasal administration of rifampicin prevented the formation of RNA foci and DPR and TDP-43 inclusions in FTD/ALS model mice. These results suggest that rifampicin at least partially prevents not only the gain-of-toxic function but also loss-of-function caused by C9orf72 HRE mutation. Regarding the mechanism by which rifampicin attenuated the pathologies state, there are several action points considered (Fig. 9). The present inventors initially speculated that rifampicin may interact with DNA/RNA to inhibit G-quadruplex formation. However, rifampicin failed to inhibit this formation. Then, it was considered that rifampicin might suppress RAN translation by inhibiting PKR phosphorylation like metformin. As a result of immunohistochemistry, administration of rifampicin significantly reduced phosphorylated PKR levels in mouse brains, supporting the hypothesis of the present inventors. This can explain why rifampicin was effective in reducing DPR and TDP-43 inclusions.

Here, C9orf72 HRE-derived RNAs are shown to form multivalent base-pairing, which causes RNA gelation without requiring protein components and leads to RNA foci formation. Notably, the RNA gelation and RNA foci formation are inhibited in vitro by, for example, doxorubicin or ammonium acetate. Since the structure of rifampicin is partially similar to the structure of doxorubicin, rifampicin may inhibit the formation of RNA foci in a similar mechanism to doxorubicin.

In neurodegenerative diseases, aggregated protein pathologies are presumed to propagate in the brain by the mechanism of cell-to-cell transmission. DPRs and TDP-43 have also been suggested to be transmitted between cells. Previously, the present inventors have demonstrated that nasal administration of rifampicin inhibits the propagation of tau oligomers in model mice. It is considered that rifampicin attenuated the neuropathology in FTD/ALS model mice by inhibiting the cell-to-cell propagation of DPRs and TDP-43.

In this example, rifampicin was administered to FTD/ALS model mice at 0.1 mg/day for 1 month. The administration of rifampicin to the mice for 1 month (31 days) corresponds to the administration to humans for about 3 years or longer. The life of a mouse is generally believed to be about 2 to about 2.2 years. For example, in Yuichi Yamashita et al., "Induction of prolonged natural lifespans in mice exposed to acoustic environmental enrichment"2018, Scientific Reports volume 8, Article number: 7909, it is described that C57BL/6J rats (male: 4, female: 4) are bred under a common experiment animal feeding environment and the average life of the rats is about 700 days (about 2 years). Meanwhile, according to "WORLD HEALTH STATISTICS OVERVIEW 2019 MONITORING HEALTH FOR THE SDGs" written by World Health Organization, it is described that the life of human is about 80 years in high-income countries. When the life of the experiment animals and the life of humans are compared, it is found that about 36 to about 40 times of the life time of the experiment animals corresponds to the life time of humans.

It is known that the expansion of short nucleotide repeats in both coding and non-coding regions underlies fifty human diseases or more. Although the causative genes and pathological phenotypes are different for each disease, it is considered that the pathological mechanism is common among them.

For example, in Huntington's disease (HD), spinocerebellar ataxia (SCA), dentatorubral-pallidoluysian atrophy, and spinal muscular atrophy, CAG repeats in the coding region expand 20 to 300 times or more, and produce a prolonged poly-Q sequence in translated proteins which then tend to self-aggregate to form inclusions and exhibit cytotoxicity. Meanwhile, nucleotide repeat expansion disorders, such as myotonic dystrophy (DM), benign adult familial myoclonic epilepsy, fragile X syndrome, fragile X-associated tremor/ataxia syndrome, and several types of SCA, possess more than or equal to 30 to 10000 times expansion of short nucleotide repeats (e.g. CTG, CCTG, TTTCA) in non-coding regions, which then generate abnormal RNAs and proteins and cause cytotoxicity via both gain-of-toxic function and loss-of-function, similar to C9orf72-linked FTD/ALS. Recently, it has been demonstrated that RAN translation also occurs in HD and that, in addition to poly-Q, four novel RAN proteins, including poly-A, poly-S, poly-L, and poly-C, are synthesized from sense and antisense repeat RNAs to accumulate in the brains. Furthermore, metformin has been shown to exhibit beneficial effects in model mice of HD and human patients with HD and DM type I.

Considering the commonality of these pathologies and the metformin-like action of rifampicin, rifampicin is believed to be effective against these coding and non-coding repeat disorders.

From the above, nasal administration of rifampicin attenuated pathological phenotypes in FTD/ALS model mice, including RNA foci, DPR and TDP-43 inclusions, neurodegeneration, and cognitive impairment. Therefore, it was shown that rifampicin had a therapeutic effect on FTD and ALS, i.e. TDP-43 proteinopathy.

### [Test Example 2]

### 1. Materials and Methods

### 1.1. Mice

FVB/NJ-Tg(C9orf72)500Lpwr/J mice (FTD/ALS model mice) were purchased from The Jackson Laboratory (Bar Harbor, ME, USA). The mouse strain was generated as a model for the C9orf72-associated FTD/ALS expressing the human C9orf72 gene with about 500 hexanucleotide (GGGGCC) repeats using a bacterial artificial chromosome (BAC) vector. Transgenic (Tg) mice were crossed with wild-type FVB/NJc1 mice, and the transgene was maintained as a heterozygote at our animal facility.

### 1.2. Administration of Rifampicin and Resveratrol

7- to 8-month-old male and female FTD/ALS model mice were divided into four groups: rifampicin alone (0.04 mg/day), resveratrol alone (0.04 mg/day), combination of rifampicin and resveratrol (0.02 mg of rifampicin + 0.02 mg of resveratrol/day), and carboxymethylcellulose (CMC) as control, based on drug administration. Rifampicin (Sigma-Aldrich, St. Louis, MO) and trans-resveratrol (Sigma-Aldrich) were dissolved in 0.5% low-viscosity CMC (Sigma-Aldrich) at 4 mg/mL. Equal amounts of rifampicin solution and resveratrol solution were mixed to form combinatorial drugs each containing 2 mg/mL of two kinds of the medicinal agents. 10 µL of rifampicin (i.e. 0.04 mg), resveratrol (0.04 mg), combinatorial drugs (0.02 mg each), or CMC solution was administered to both nasal passages using a microchip 5 days per week, from Monday to Friday, for 1 month. Age-matched non-Tg rat mates received only CMC.

### 1.3. Behavioral Test

After administration for one month, the cognitive function of the mice was assessed in the Morris water maze test as described above.

### 1.4. Immunohistochemical Analysis

After the behavioral test, each group was divided into two groups: one for immunohistochemical analysis and the other for future biochemical analysis. Immunohistochemical staining was performed as described above. For staining of double-stranded RNA-dependent protein kinase (PKR), phosphorylated TDP-43, synaptophysin, sections were boiled in 10 mM citrate buffer solution (pH6) for 30 min to expose the antigens. The sections were blocked overnight with 10% calf serum, and then the resultant sections were stained with antibodies for DNA/RNA G-quadruplex (BG4; Absolute antibody, Cleveland, UK), phospho-PKR (Thr446) (MilliporeSigma, Burlington, MA, USA), poly-GA, poly-GP (Cosmo Bio Co., Ltd., Tokyo, Japan), pSer409/410-TDP-43 (Cosmo Bio Co., Ltd.), and synaptophysin (SVP-38; Sigma-Aldrich, St. Luis, MO, USA). Only synaptophysin was stained with a biotin-labeled secondary antibody (Vector Laboratories, Burlingame, CA, USA), a horseradish peroxidase (HRP) -labeled avidin-biotin complex (Vector Laboratories), HRP substrate, diaminobenzidine (DAB), or an FITC-labeled secondary antibody (The Jackson Laboratory). The stained specimens were observed with a fluorescence microscope BZ-X800 (KEYENCE CORPORATION, Osaka, Japan), and images of specific brain regions were taken. Neuropathologies were evaluated by measuring the staining intensity or area or by counting positive puncta or cells in a constant area in each photograph using NIH ImageJ software.

### 1.5. Statistical Analysis

All experiments and data analyses were performed in an unblinded manner. A comparison of average values among two groups or more was performed by ANOVA or two-factor repeated measurements ANOVA (for the Morris water maze test), followed by Fisher's PLSD test. Differences with a p-value < 0.05 were considered significant.

### 2. Results

Nasal administration of resveratrol (Resv) 0.04 mg/day significantly improved the cognitive function of FTD/ALS model mice (Fig. 10). Administration of the same dose of rifampicin (RFP) also improved the cognitive function in the mice, but the effect was incomplete and not significant. The combination of rifampicin and resveratrol (0.02 mg of rifampicin + 0.02 mg of resveratrol/day) showed an intermediate effect between resveratrol and rifampicin.

Synaptophysin levels were evaluated in the hippocampus CA2-3 region and the dentate gyrus (DG). Administration of resveratrol significantly restored synaptic loss in the CA2-3 region and the DG, but administration of rifampicin did not restore synaptic loss (Fig. 11). The combination of resveratrol and rifampicin was effective only in the DG. In the prefrontal cortex, the levels of G-quadruplex (Fig. 12), phosphorylated PKR (Fig. 13), poly-GA and poly-GP (Fig. 14), phosphorylated TDP-43 (Fig. 15) were evaluated. Rifampicin, resveratrol, and a combination of rifampicin and resveratrol significantly reduced these pathologies, resveratrol showed the strongest effect, and the combination showed an intermediate effect between resveratrol and rifampicin.

### 3. Discussion

In this study, rifampicin, resveratrol, and a combination of rifampicin and resveratrol improved cognition and C9orf72 hexanucleotide repeat expansion (HRE) - related neuropathology in FTD/ALS model mice, and resveratrol showed a stronger effect than rifampicin. The difference in the effect between rifampicin and resveratrol is considered to be due to the difference in the action mechanism. Both rifampicin and resveratrol have an antioxidant action and are effective in forming amyloid oligomers. We have previously shown that rifampicin inhibits the oligomerization of Aβ and tau in model mice more efficiently than resveratrol, suggesting that rifampicin can inhibit the oligomerization of TDP-43 more efficiently than resveratrol. However, in C9orf72-linked FTD/ALS, TDP-43 oligomerization occurs downstream of the pathological cascade, and its suppression may show only a partial therapeutic effect. Upstream pathology: Changes in DNA/RNA structure caused by HRE leading to, for example, C9orf72 haploinsufficiency, RNA foci formation, RAN translation, stress granule formation can cause more extensive and serious damage to cells, which indicates that the improvement effect of resveratrol is higher than that of rifampicin. The expression of BDNF in the mouse brains was elicited by resveratrol, but not by rifampicin. Furthermore, resveratrol alone has been reported to activate Sirt1, i.e. a gene associated with longevity. It is considered that these resveratrol activities contribute to a stronger effect on HRE-related neuropathologies.

The present results suggest that resveratrol is a promising therapeutic candidate for FTD/ALS.

### 4. Conclusions

Nasal administration of rifampicin (single agent) at 0.04 mg/day, resveratrol (single agent) at 0.04 mg/day, and a combination of rifampicin at 0.02 mg/day and resveratrol at 0.02 mg/day improved neuropathological and cognitive dysfunction in FTD/ALS model mice. Therefore, it was shown that rifampicin and resveratrol had a therapeutic effect on FTD and ALS, i.e. TDP-43 proteinopathies. In addition, resveratrol showed the strongest effect, and the combination showed an intermediate effect between resveratrol and rifampicin, indicating that resveratrol has a particularly high therapeutic effect on FTD and ALS: TDP-43 proteinopathies. Therefore, even in a case the dose of resveratrol (single agent) is half the above-described dose (specifically, 0.02 mg/day), of course, the improvement of neuropathological and cognitive dysfunction and further the therapeutic effect on FTD and ALS: TDP-43 proteinopathies can be inferred.

## Claims

1. A preventive or therapeutic agent for a neurodegenerative disease caused by accumulation of TDP-43, comprising, as an active ingredient, a resveratrol compound selected from the group consisting of resveratrol and a derivative of resveratrol.

2. A preventive or therapeutic agent for amyotrophic lateral sclerosis (ALS), comprising, as an active ingredient, a resveratrol compound selected from the group consisting of resveratrol and a derivative of resveratrol.

3. A preventive or therapeutic agent for a neurodegenerative disease caused by C9orf72 gene abnormality and/or TDP-43 gene abnormality, comprising, as an active ingredient, a resveratrol compound selected from the group consisting of resveratrol and a derivative of resveratrol.

4. The preventive or therapeutic agent according to any one of claims 1 to 3, wherein the preventive or therapeutic agent is used for nasal administration.

5. The preventive or therapeutic agent according to any one of claims 1 to 3, wherein a dose of the resveratrol compound is 3.75 mg/kg·day or less.

6. A preventive or therapeutic agent for a neurodegenerative disease caused by accumulation of TDP-43, comprising a combination of a rifampicin compound selected from the group consisting of rifampicin, a derivative of rifampicin, and a salt of rifampicin or the derivative, and a resveratrol compound selected from the group consisting of resveratrol and a derivative of resveratrol.

7. A preventive or therapeutic agent for a neurodegenerative disease caused by accumulation of TDP-43, comprising, as an active ingredient, a rifampicin compound selected from the group consisting of rifampicin, a derivative of rifampicin, and a salt of rifampicin or the derivative.

8. A preventive or therapeutic agent for amyotrophic lateral sclerosis (ALS), comprising, as an active ingredient, a rifampicin compound selected from the group consisting of rifampicin, a derivative of rifampicin, and a salt of rifampicin or the derivative.

9. A preventive or therapeutic agent for a neurodegenerative disease caused by C9orf72 gene abnormality and/or TDP-43 gene abnormality, comprising, as an active ingredient, a rifampicin compound selected from the group consisting of rifampicin, a derivative of rifampicin, and a salt of rifampicin or the derivative.

10. The preventive or therapeutic agent according to any one of claims 7 to 9, wherein the preventive or therapeutic agent is used for nasal administration.

11. The preventive or therapeutic agent according to any one of claims 7 to 9, wherein a dose of the rifampicin compound is 3.75 mg/kg·dayor less.
